# EUROPEAN PATENT SPECIFICATION

(11) **EP 3 492 449 B1**
(45) Date of publication and mention of the grant of the patent: **23.09.2020**
(21) Application number: 17306691.1
(22) Date of filing: 01.12.2017
(51) Int. Cl.: C07C 45/66, C07C 45/74, C07D 493/08, C08G 65/40

(54) **PROCESS FOR THE PREPARATION OF DIHALOBENZOPHENONES, NEW CHEMICALS USEFUL FOR ITS IMPLEMENTATION AND METHODS FOR PREPARING SAID CHEMICALS**
VERFAHREN ZUR HERSTELLUNG VON DIHALOBENZOPHENONEN, NEUE, ZU DESSEN DURCHFÜHRUNG NÜTZLICHE CHEMIKALIEN UND VERFAHREN ZUR HERSTELLUNG BESAGTER CHEMIKALIEN
PROCÉDÉ POUR LA PRÉPARATION DE DIHALOBENZOPHÉNONES, NOUVEAUX PRODUITS CHIMIQUES UTILES POUR LEUR MISE EN OEUVRE ET PROCÉDÉS DE PRÉPARATION DESDITS PRODUITS CHIMIQUES

(43) Date of publication of application: 05.06.2019
(73) Proprietor: Rhodia Operations, 93300 Aubervilliers (FR)
(72) Inventor: MULLER, Eric, 69003 LYON (FR)
(74) Representative: Delenne, Marc

(56) References cited:
- WO-A1-2012/001131
- WO-A1-2017/096559
- PIERRE LASZLO ET AL: "Easy formation of diels-alder cycloadducts between furans and [alpha],[beta]-unsaturated aldehydes and ketones at normal pressure.", TETRAHEDRON LETTERS, vol. 25, no. 39, 1 January 1984 (1984-01-01), pages 4387-4388, XP055477874, AMSTERDAM, NL ISSN: 0040-4039, DOI: 10.1016/S0040-4039(01)81445-3

## Description

### TECHNICAL FIELD

The present invention pertains to new and useful specific chemical compounds which are obtainable by cycloaddition between either a (vinyl)(hydrocarbyl)ketone or a (vinyl)(phenyl)ketone and a halofuran (Diels-Alder reaction) and to their use as precursors and/or intermediates for the preparation of dihalobenzophenones.

### BACKGROUND ART

Dihalobenzophenones are valuable chemical compounds which are known to the art. They are mainly prepared by oxidation of the methylene group of a dihalodiphenyl methane to provide the corresponding dihalobenzophenone. Various other methods are known, such as those described in US 4943358, WO 2012/001131, US 5777172 and in Dunlop et al., "The preparation of 4-fluoro- and 4,4'-difluorobenzophenone", J. Am. Chem. Soc., 1933, 55(4), pp. 1665-1666. A non-exhaustive summary of other available processes for the manufacture of 4,4'-difluorobenzophenone may also be found in US 7687668.

4,4'-difluorobenzophenone (4,4'-DFBP) is the central starting material for preparing poly(aryl ether ketone)s (PAEK) which are a well-known class of engineering polymers used in various fields. These are high-performance polymers with constantly growing annual production volumes, so that the volume of 4,4'-DFBP produced worldwide annually is also included in the growth. The most important polyether ketones are the poly(ether ketone)s (PEK) and poly(ether ether ketone)s (PEEK), which feature melting points of above 330°C and high chemicals resistance.

The present invention aims at providing a new and efficient process for the preparation of dihalobenzophenones.

Another objective of the present invention is to provide a process for the preparation of dihalobenzophenones involving renewable starting materials.

### SUMMARY OF THE INVENTION

A first object of the present invention relates to a process for the preparation of a 4,4'-dihalobenzophenone of formula (I): 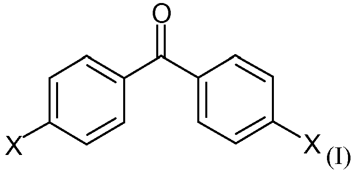 wherein X represents a halogen atom selected from the group consisting of fluorine, chlorine, bromine and iodine,
said process comprising the steps of:
a1) reacting a halofuran of formula (II) with a vinylhydrocarbylketone of formula (III): 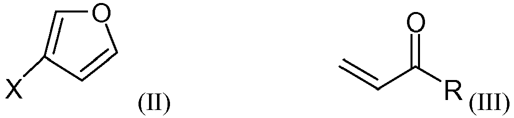 wherein X is the same as defined above, and R is a methyl radical,
   thereby providing reaction products containing a cycloadduct compound of formula (IV): 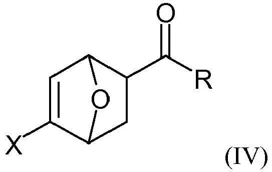 in which X and R are the same as defined above
a2) optionally further isolating said compound of formula (IV)
b) carrying out a dehydration/aromatization of said adduct compound of formula (IV), thereby providing reaction products containing a (p-halogeno)phenyl hydrocarbyl ketone of formula (V): 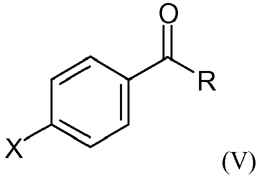 in which X and R are the same as defined above
c) carrying out an alpha-methylenation of said compound of formula (V), thereby providing reaction products containing a (p-halogeno)phenyl vinyl ketone of formula (VI): 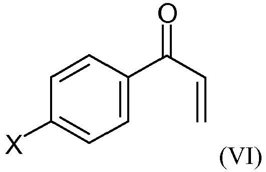 in which X is the same as defined above
d1) reacting said compound of formula (VI) with the same halofuran of formula (II) as defined above, thereby providing reaction products containing a cycloadduct compound of formula (VII): 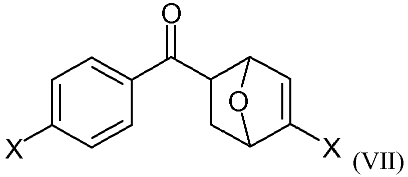 in which X is the same as defined above
d2) optionally further isolating said compound of formula (VII)
e) carrying out a dehydration/aromatization of said adduct compound of formula (VII), thereby providing reaction products containing said 4,4'-dihalobenzophenone of formula (I).

The invention also relates to the compounds of formula (IV) and (VII) as defined above, which are indeed chemicals new per se.

The invention also pertains with a process for the manufacture of said compounds of formula (IV) and (VII), said process involving in both cases a Diels-Alder reaction between a halofuran and a monovinylketone.

The invention further relates to the use of a compound of formula (VII) as defined above, for the direct preparation of a 4,4'-dihalobenzophenone of formula (I), said preparation involving a dehydration/aromatization reaction step on a compound of formula (VII).

According to the invention, in all its aspects, the halogen atom X is preferably selected from bromine and chlorine, more preferably chlorine.

According to the invention, in all its aspects, the hydrocarbyl radical R may be chosen among the alkyl radicals or the aryl radicals. Preferably, R is an alkyl radical, linear or branched, substituted or unsubstituted, which may contain from 1 to 20 carbon atoms. Most preferably, R is an unsubstituted linear C1-C10 alkyl radical, even most preferably an unsubstituted linear C1-C2 alkyl radical.

According to the invention, in all its aspects, the Diels-Alder reactions may be carried out with or without the use of a solvent, and with or without the use of a catalyst.

According to an embodiment of the invention, in all its aspects, the dehydration/aromatization reactions are preferably carried out in the presence of an alkali methoxide, preferably sodium methoxide or potassium methoxide, or an alkali hydroxide, preferably sodium hydroxide or potassium hydroxide.

According to another embodiment of the present invention, the dehydration/aromatization reactions are preferably carried out in the presence of a solvent. The solvent is advantageously chosen from alcohols, sulfoxides and mixtures thereof. It is preferably chosen from C₁-C₆ (especially C₁-C₄) aliphatic alcohols, cyclohexanol, dialkyl sulfoxides wherein each out of the 2 alkyl groups contains from 1 to 6 carbon atoms (especially from 1 to 4 carbon atoms), alkyl phenyl sulfoxides wherein the alkyl group contains from 1 to 6 carbon atoms (especially from 1 to 4 carbon atoms), tetrahydrothiophene 1-oxide and mixtures thereof. Good results were obtained notably when using dimethyl sulfoxide (DMSO) as the solvent.

As already mentioned, one of the advantages of the invention is to provide a process for the preparation of 4,4'-dihalobenzophenones involving renewable starting materials.

Moreover, it has been surprisingly found that the process for the preparation of compound of formula (I) according to the invention allows satisfactory conversion and selectivity.

### DETAILED DESCRIPTION OF THE INVENTION

The rational of the different chemicals (some being new per se) and chemical steps involved in the present invention may be summarized as follows (in this scheme, R is methyl, but could also represent another hydrocarbyl radical as detailed hereinafter): 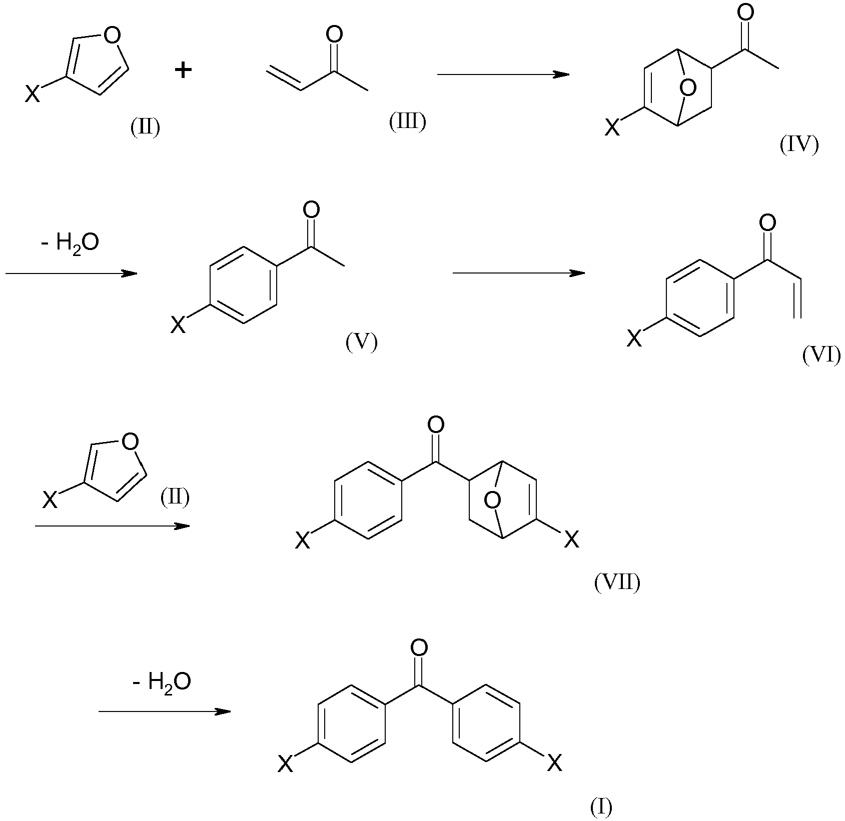 in which -H₂O symbolizes a dehydration/aromatization step carried out on a cycloadduct.

Preferably, the halofuran of formula (II) is 3-chlorofuran or 3-bromofuran.

The halofuran of formula (II) can be obtained from furan according to processes known to the person skilled in the art. Document US 2,773,882 describes such a process.

Furan may be prepared by decarbonylation of furfural which is obtained from a renewable resource, see for example US 4,764,627 and US 8,754,245. Suitable renewable resources as well as suitable methods for their conversion into furfural are known to the person skilled in the art.
Alternatively, the halofuran of formula (II) may be prepared by any other conventional chemical reactions, or may be also commercially available products.

The vinylhydrocarbylketone of formula (III) can be obtained from the corresponding hydrocarbylketone and formaldehyde, for example as described by Siegel H. and Eggersdorfer M., Ullmann's Encyclopedia of Industrial Chemistry (2000). Moreover, some of these products, in particular methylvinylketone, are commercially available.

It has been surprisingly found that a Diels-Alder reaction (step a1) efficiently occurs between the halofuran of formula (II) and the vinylhydrocarbylketone of formula (III) resulting in the formation of a cycloadduct of formula (IV) as defined above. Moreover, it has been surprisingly found that said Diels-Alder reaction leads to the formation of compounds of formula (IV) with a good conversion, a low amount of byproducts and a good selectivity in the desired product.

The present invention therefore also provides a process for the production of compound of formula (IV), comprising reacting a halofuran of formula (II) with a vinylhydrocarbylketone of formula (III), said two formulae being the same as defined above, and optionally further isolating compound of formula (IV).

As already mentioned, compounds of formula (IV) are new and useful products per se, and are therefore part of the present invention.

The Diels-Alder reaction between the halofuran of formula (II) and the vinylhydrocarbylketone of formula (III) leads to the formation of two main structural isomers represented below, one of which being the compound of formula (IV):

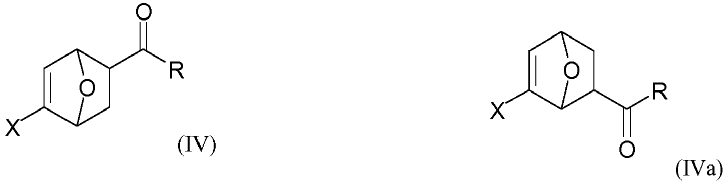

The reaction between compound of formula (II) and compound of formula (III) might also lead to the formation of byproducts in minor amounts, in particular the compound of formula (A):

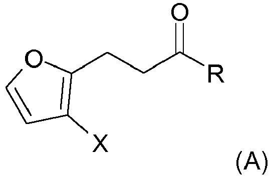

According to an embodiment of the invention, the molar ratio of compounds of formula (IV) to the compounds of formula (IVa) is of at least 50/50, preferably at least 70/30, more preferably at least 80/20, even more preferably at least 90/10.

According to an embodiment of the invention, the molar ratio of compound of formula (IV) to compound of formula (A) is of at least 50/50 or at least 60/40 or at least 80/20 or at least 90/10, and even 100/0.

According to an embodiment of the invention, the conversion of the Diels-Alder reaction is of at least 50%, preferably at least 70%, more preferably at least 90%.

The Diels-Alder reaction between the halofuran of formula (II) and the vinylhydrocarbyl ketone can be carried out under usual Diels-Alder conditions known to the person skilled in the art.

The Diels-Alder reaction may be conducted with or without a catalyst. The use of catalysts can improve kinetics and selectivity of the Diels-Alder reaction. Known Diels-Alder catalysts may be used and may include Lewis acids, such aluminium chloride, ethylaluminium dichloride, diethylaluminium chloride, trimethyl aluminium, bismuth (III) chloride, bismuth (III) trifluoromethanesulfonate, boron trifluoride, boron triacetate, cerium (III) chloride, copper (I) trifluoromethanesulfonate, copper (II) chloride, hafnium (IV) chloride, iron (II) chloride, iron (II) acetate, iron (III) chloride, iron (III) acetate, lithium perchlorate, lithium trifluoromethanesulfonate, magnesium bromide, magnesium iodide, magnesium chloride, magnesium perchlorate, scandium (III) trifluoromethanesulfonate, tin (IV) chloride, titanium (IV) chloride, titanium (IV) isopropoxide, N-trimethylsilyl-bis(trifluoromethanesulfonyl)imide, trimethylsilyl trifluoromethanesulfonate, ytterbium (III) trifluoromethanesulfonate, zinc chloride, zinc bromide, zinc iodide, zinc acetate zirconium (IV) chloride and indium (III) chloride, triphenylborane, Bronsted acids, such as inorganic mineral acids, e.g. sulphuric acid, phosphoric acid, nitric acid, hydrobromic acid or hydrochloric acid, and organic acids such as methane sulphonic acid, p-toluenesulphonic acid or carboxylic acids. One of the preferred Diels-Alder catalysts is magnesium iodide.

Alternatively, activated carbon, silica, alumina, silica-alumina, zirconia and zeolites may be used as such or as support for a catalytically active metal or metal compound. Suitable metals or metal compounds include alkali metals, alkaline earth metals, transition metals, noble metals, rare earth metals. The catalysts can be acidic, e.g. by treating supports with phosphoric acid, or by ion exchange of zeolites to render them into their acidic form. Examples of solid catalysts include amorphous silica-alumina, zeolites, preferably zeolites in their H-form, and acidic ion exchange resins. Other suitable catalysts that are liquids or that may be dissolved in the appropriate solvent to yield a homogeneous catalyst environment, include organic and inorganic acids, such as alkane carboxylic acid, arene carboxylic acid, sulphuric acid, phosphoric acid, hydrochloric acid, hydrobromic acid and nitric acid.

The Diels-Alder reaction may be carried out with or without a solvent. Suitable solvents may be selected from pyridine, tertiary amines such as triethylamine and diisopropylethylamine, chloroform, dichloromethane, diethyl ether, perfluorinated alkanes such as perfluorohexane, toluene, water and ionic liquids such as 1-butyl-3-methylimidazolium tetrafluoroborate and 1-butyl-3-methylimidazolium hexafluorophosphate, alone or in a mixture. Preferred solvents are pyridine and tertiary amines, such as triethylamine. In case a catalyst is used, it is preferred to use dichloromethane as solvent or to use no solvent at all.

The Applicant has also surprisingly noticed that, under solvent conditions, certain basic additives such as pyridine and tertiary amines, such as triethylamine, stabilize the reactants which allows an excess of the halofuran of formula (II) to be used in order to increase the conversion of the Diels-Alder reaction. The excess of the halofuran used may be recovered once the reaction is completed.

The reaction may be carried out at any suitable temperature, for example from about -80 to about 120 °C, preferably from about -20 to about 100 °C, more preferably from about -10 to about 80 °C, for a time sufficient to convert the starting compounds into the desired Diels-Alder adduct, such as about 10 minutes to about 6 days, preferably about 3 hours to about 4 days, more preferably about 3 hours to about 2 days. The reaction can be carried out at ambient pressure or under increased pressure. In a preferred embodiment, the reaction is carried out at ambient pressure, such as about 1 bar, or at a pressure of up to 10 bars, preferably up to 5 bars, more preferably up to 2 bars.

According to an embodiment of the invention, the molar ratio of the halofuran of formula (II) to vinylhydrocarbylketone of formula (III) is close to stoichiometry (namely 1/1), or above.

The Applicant has found that a molar ratio of the halofuran of formula (II) to the vinylhydrocarbylketone of formula (III) greater than 1/1 allows for the displacement of the Diels-Alder reaction towards the formation of the cycloadduct of formula (IV).

The process according to the invention may comprise a further step of isolation of compound of formula (IV). Suitable methods of isolation and purification are known to the person skilled in the art. For example, chromatography techniques, such as liquid chromatography, may be particularly used in the process according to the invention.

Alternatively, the mixture obtained at the end of the Diels-Alder reaction and comprising structural isomers and eventually byproducts can be directly used in the subsequent step of dehydration/aromatization explained below.

In a subsequent step (step b) according to the invention, a dehydration/aromatization reaction is conducted on the compound of formula (IV) in order to provide a (para-halogeno)phenyl hydrocarbyl ketone of formula (V).

The reaction conditions for aromatization of the compound of formula (IV) are known to a person skilled in the art.

According to an embodiment of the invention, the aromatization reaction of the compound of formula (IV) requires basic reaction conditions, for example in the presence of an alkoxide compound or hydroxide compound. Suitable alkoxides may be selected from alkali methoxides, such as sodium methoxide and potassium methoxide, preferably sodium methoxide. Suitable hydroxides may be selected from alkali hydroxides, such as sodium hydroxide, potassium hydroxide, lithium hydroxide, cesium hydroxide, preferably sodium hydroxide and potassium hydroxide, more preferably sodium hydroxide.

Suitable solvents for the aromatization reaction may be for example a sulfoxide such as DMSO or an alcohol such as isopropanol.

The aromatization reaction may be carried out at any suitable temperature, for example from about -10 to about 120 °C, preferably from about -5 to about 80 °C, more preferably from about 0 to about 40 °C.

As another subsequent step (step c) according to the invention, the compound of formula (V) is alpha-methylenated in order to provide a (para-halogeno) phenyl ketone of formula (VI):

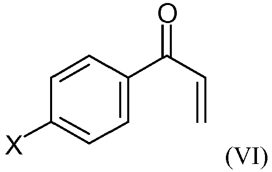

Alpha-methylenation is a known reaction for the one skilled in the art. For example, it may be implemented by heating (60 to 80 °C) the compound (V) in an aprotic solvent like THF or MeTHF in the presence of a formaldehyde source (paraformaldehyde, trioxane...) and a catalyst (an acid salt of a secondary amine) (see Chem. Comm., 2010, 46, pp. 1715-1717).

In the case the radical R of the compound of formula (V) is ethyl, an alternative to the alpha-methylenation reaction is to perform a dehydrogenation reaction on said compound which will lead to the same product of formula (VI).

Once the compound of formula (VI) has been obtained, a second Diels-Alder reaction is conducted either directly on the reaction products prepared in the previous step and containing the compound (VI), or on the sole compound (VI) after separation thereof from the reaction products.

The conditions to implement said reaction may be, and preferably are, the same as, or similar to, the ones that were used in the first Diels-Alder reaction (step a1) detailed above. Most importantly, the use of the same halofuran of formula (II) must be respected.

The implementation of said second Diels-Alder reaction will lead to the formation of a second cycloadduct of formula (VII):

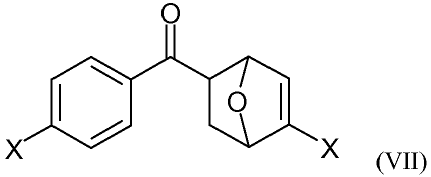

As already mentioned, compounds of formula (VII) are new and useful products per se, and are therefore also part of the present invention.

The present invention therefore also provides a process for the production of compound of formula (VII), comprising reacting a halofuran of formula (II) with a (para-halo)phenyl vinyl ketone of formula (VI), said two formulae being the same as defined before, and optionally further isolating compound of formula (VII).

Again, it has been surprisingly found that a Diels-Alder reaction efficiently occurs between the halofuran of formula (II) and the (para-halo)phenyl vinyl ketone of formula (VI) resulting in the formation of a new cycloadduct of formula (VII) as defined above. Moreover, it has been surprisingly found that said Diels-Alder reaction leads to the formation of compounds of formula (VII) with a good conversion, a low amount of byproducts and a good selectivity in the desired product.

The Diels-Alder reaction between the halofuran of formula (II) and the (para-halo)phenyl vinyl ketone of formula (VI) leads to the formation of two main structural isomers represented below, one of which being the compound of formula (VII):

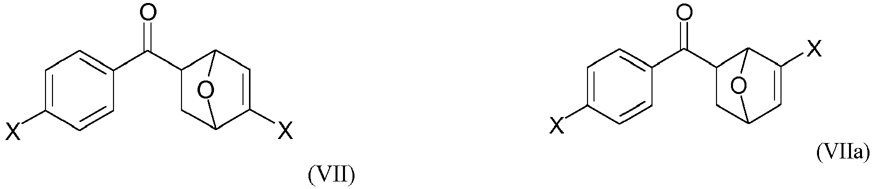

According to an embodiment of the invention, the molar ratio of compounds of formula (VII) to the total of compounds of formulae (VIIa) is of at least 50/50, preferably at least 70/30, more preferably at least 80/20.

According to an embodiment of the invention, the conversion of the second Diels-Alder reaction is of at least 50%, preferably at least 70%, more preferably at least 90%.

According to an embodiment of the invention, the molar ratio of the halofuran of formula (II) to (para-halo)phenyl vinyl ketone of formula (VI) is close to stoichiometry (namely 1/1), or above.

Like for the process for the preparation of the cycloadduct compound of formula (IV), the process according to the invention may comprise a further step of isolation of compound of formula (VII). Suitable methods of isolation and purification are known to the person skilled in the art. For example, chromatography techniques, such as liquid chromatography, may be particularly efficient in the process according to the invention.

At last, owing to a final dehydration/aromatization reaction conducted on said compound of formula (VII) (step e), the desired 4,4'-dihalobenzophenone will be ultimately obtained.

The conditions to implement said final dehydration/aromatization reaction may be, and preferably are, the same as, or similar to, the ones that were used in the first dehydration/aromatization reaction (step b) already detailed above.

The present invention also relates to a process for the direct preparation of a 4,4'-dihalobenzophenone of formula (I): 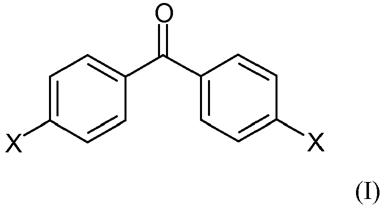 wherein X represents a halogen atom selected from the group consisting of fluorine, chlorine, bromine and iodine, comprising carrying out the dehydration/aromatization of the compound of formula (VII) as defined above.
Preferably, X is selected from fluorine and bromine, more preferably fluorine.

Another object of the invention relates to a process for producing a 4,4'-dihalobenzophenone of formula (I): 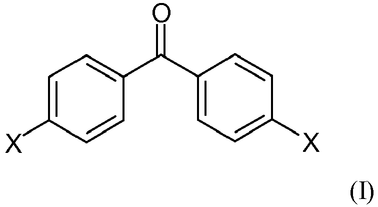 wherein X represents a halogen atom selected from the group consisting of fluorine, chlorine, bromine and iodine,
comprising:
a) providing a compound of formula (VII) as defined above,
b) carrying out the dehydration/aromatization of said compound of formula (VII).

Alternatively, 4,4'-difluorobenzophenone may be prepared by effecting a halogen-fluorine exchanging reaction between compound of formula (I) wherein X is chlorine, bromine or iodine, and an alkali fluoride, such as potassium fluoride, as described in US 4,453,009.

According to an embodiment of the invention, 4,4'-dihalobenzophenone of formula (I) is further isolated by separation methods known to the person skilled in the art. For example, recrystallization may be carried out according to the method described in document US 4,873,372.

Poly(aryl ether ketone)s (PAEK) are a well known class of engineering polymers useful in various fields of scientific and commercial endeavour. PAEK polymers are generally prepared by aromatic nucleophilic substitution of both the halogenides of a 4,4'-dihalobenzophenone (especially, by aromatic nucleophilic substitution of both the fluorides of 4,4'-difluorobenzophenone) by a nucleophilic oxygen atom from a co-monomer. For example, p-hydroquinone, commonly referred to as "hydroquinone" and/or at least one bisphenol (such as bisphenol A, bisphenol S, bisphenol O, 4,4'-dihydroxybiphenyl and mixtures thereof) can be used as the co-monomer; the hydrogen atom from each of the two aromatic hydroxyl groups of p-hydroquinone and/or of the bisphenol is advantageously deprotonated with at least one base (such as NaOH, Na₂CO₃, K₂CO₃ and mixtures thereof) to form a nucleophile which reacts with the 4,4'-dihalobenzophenone (preferably, 4,4'-difluorobenzophenone) to form a PAEK polymer via a nucleophilic substitution mechanism, with the halogen atoms of the 4,4'-dihalobenzophenone acting as leaving groups. Processes for preparing PAEK polymers, including those using a 4,4'-dihalobenzophenone such as 4,4'-difluorobenzophenone, can be found notably in U.S. Pat. Nos. 3,953,400, 3,956,240, 3,928,295, and 4,176,222.

Then, facets of the present invention relate to :
- the use of a compound of formula (VII) as previously defined for the manufacture of a poly(aryl ether ketone), in particular poly(ether ether ketone), typically with a 4,4'-dihalobenzophenone of formula (I) as synthesis intermediate,
- the use of a compound of formula (IV) as previously defined wherein R is a methyl radical for the manufacture of a poly(aryl ether ketone), in particular poly(ether ether ketone), typically with a 4,4'-dihalobenzophenone of formula (I) as synthesis intermediate, and

Further disclosed is a process for the manufacture of a (poly aryl ether ketone), said process comprising the following steps:
i) providing a compound of formula (VII) as defined above,
ii) carrying out the dehydration/aromatization of said compound of formula (VII) to prepare a 4,4'-dihalobenzophenone of formula (I) as defined above,
iii) optionally, isolating the 4,4'-dihalobenzophenone,
iv) optionally, effecting a halogen-fluorine exchanging reaction between the 4,4'-dihalobenzophenone and an alkali fluoride as explained above ,
v) carrying out the polycondensation of the 4,4'-dihalobenzophenone with at least one aromatic compound comprising 2 hydroxyl groups, in particular with hydroquinone and/or at least one bisphenol, preferably in the presence of at least one base.

The present invention will now be illustrated by the following examples, which are not intended to be limiting.

### EXAMPLES

### Example 1: Preparation of 4,4'-dibromobenzophenone (compound of formula (I)) from 4'-bromoacetophenone (compound of formula (V))

### 1/ Preparation of 4'-bromoacetophenone

In a carousel tube fitted with a PTFE septum screw cap methylvinylketone (190 mg, 2.7 mmol) was weighted. Then, 3-bromofuran (2.0 g, 13.6 mmol) and diisopropylethylamine (50 mg) were added. The reaction mixture was stirred at 60 °C during 48 h. ¹H NMR analysis shows a 87% conversion of methylvinylketone to Diels-Alder adducts. For example, one of the isomers of Diels-Alder adducts have the following NMR signature: ¹H NMR (400 MHz, DMSO-d6) δ: 6.55 (d, J=2.0 Hz, 1H), 5.15 (m, 1H), 4.85 (d, J=4.8 Hz, 1H), 2.68 (dd, J=8.4, 4.0 Hz, 1H), 2.19 (s, 3H), 1.98 (dt J=11.2, 4.4 Hz, 1H), 1.52 (dd, J=11.6, 8.4 Hz, 1H).

DMSO (0.75 mL) was added to the reaction media and the mixture was transferred at 20°C in a Schlenk tube under argon atmosphere containing powdered sodium hydroxide (47 mg, 1.18 mmol). The reaction mixture was stirred at 40 °C during 23 h. GC titration of the final reaction mixture showed the formation of 175 mg of 4'-bromoacetophenone (37% yield).

### 2/ Alpha-methylenation of 4'-bromoacetophenone

In a 250 ml triple-neck round-bottom flask with condenser and magnetic stirring bar, 4'-bromoacetophenone (5.0 g; 25.1 mmol) was weighted. Then, MeTHF (62.8 mL) and diisopropylamine (5.2 mL, 37.6 mmol) were added. The mixture was cooled to 10°C and trifluoroacetic acid (1.9 mL, 25.1 mmol) was added in 5 minutes. Then paraformaldehyde (2.26 g, 75.4 mmol) was added and the mixture was heated at 70°C during 16 h.

After cooling, water (50 mL) was added and the product is extracted with dichloromethane (3x50 mL). The combined organic layers were dried over MgSO₄ and concentrated *in vacuo* to afford 6.9 g of crude product.

This product was purified by silica gel chromatography (cyclohexane/ethyl acetate) to afford 3.1 g of 4'-bromoacrylophenone (compound of formula (VI)).

### 3/ Diels-Alder reaction with 3-bromofuran

In a carousel tube fitted with a PTFE septum screw cap 4'-bromoacrylophenone (462 mg, 2.2 mmol) was weighted. Then, 3-bromofuran (2.1 g, 14.2 mmol) and diisopropylethylamine (50 mg) were added. The reaction mixture was stirred at room temperature during 27 h. ¹H NMR show a 97% conversion of 4'-bromoacrylophenone to Diels-Alder adducts.

### 4/ Aromatization of the Diels-Alder adducts

DMSO (1.5 mL) was added to the crude reaction media from the previous example and the mixture was transferred in a Schlenk tube under argon atmosphere containing powdered sodium hydroxide (55 mg, 1.4 mmol). The reaction mixture was stirred at 40°C during 3 h. GC titration of the final reaction mixture showed the formation of 127 mg of 4,4'-dibromobenzophenone and 10 mg of 3,4'-dibromobenzophenone, corresponding to a yied of 19 % in dibromobenzophenones and a 4,4' / 3,4' ratio of 93/7.

### Example 2: influence of a solvent on the Diels-Alder reaction (step a1)

### Protocol:

In a carousel tube fitted with a PTFE septum screw cap ethylvinylketone (143 mg, 1.7 mmol) was weighted. Then, 3-bromofuran (500 mg, 3.4 mmol) and the indicated solvent (0.5 mL) were added. The reaction mixture was stirred at 60°C during 20 h. No catalyst is used. The conversion and the selectivity to Diels-Alder adducts are analyzed by NMR.
NMR (DMSO-d6, δ ¹H [δ ¹³C]):

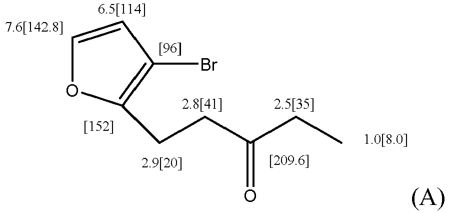

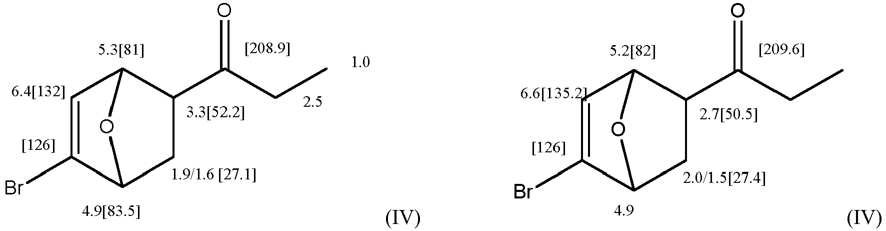

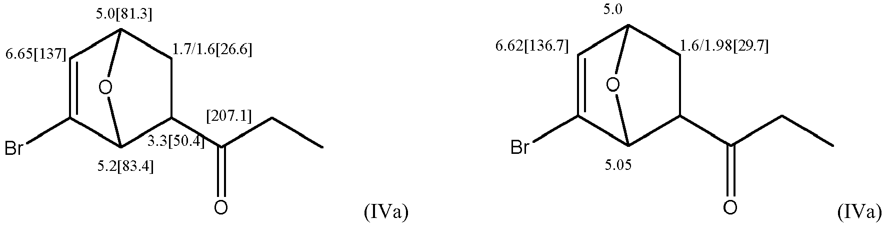

The para adducts of formula (IV) are present as exo or endo isomers. The same applies to the meta adducts of formula (IVa).

### Results:

**Table 1**

| Solvent | TR (%) | Molar ratio X/A |
|---|---|---|
| no solvent added | 94 | 47/43 |
| Diethylether (50 wt%) | 84 | 85/15 |
| DCM (50 wt%) | 85 | 75/25 |
| Pyridine (50 wt%) | 65 | 100/0 |
| DIPEA (50 wt%) | 56 | 100/0 |
| DIPEA (1 drop) | 80 | 100/0 |

TR: transformation rate
X: total amount of the four isomers (IV) and (IVa)
DCM: dichloromethane
DIPEA: diisopropylethylamine

### Example 3: influence of a catalyst on the Diels-Alder reaction (step a1)

### Protocol:

In a carousel tube fitted with a PTFE septum screw cap the catalyst (0.5 mmol) was weighted and dichloromethane (3.5 mL) was added. The reaction mixture was cooled to the indicated temperature and the ethylvinylketone (286 mg, 3.4 mmol) and 3-bromofuran (500 mg, 3.4 mmol) were added. The reaction mixture was stirred at the indicated temperature during 5 h.

Then a saturated aqueous solution of sodium carbonate (2.5 mL) was then added and the mixture was allowed to warm to room temperature.

The conversion and the selectivity to Diels-Alder adducts were estimated by NMR analysis of the organic phase.

The composition of the reaction products is given in table 2.

**Table 2**

| | | Reactants (mol%) | | Products (mol%) | | Regioselectivity of the Diels-Alder adducts IV (mol%) | | | |
|---|---|---|---|---|---|---|---|---|---|
| | | 3-bromofuran | ethylvinyl ketone | X | A | meta (IVa) | | para (IV) | |
| AlCl₃ | -40°C, 15 min | 52 | 0 | 39 | 9 | 13 | 36 | 33 | 18 |
| AlCl₃ | -78°C, 5h | 14 | 6 | 80 | 0 | 5 | 1 | 59 | 35 |
| HfCl₄ | -78°C, 5h | 8 | 2 | 88 | 2 | 4 | 5 | 65 | 26 |
| BF₃Et₂O | -78°C, 5h | 17 | 4 | 69 | 10 | low resolution NMR | | | |
| MgI₂ | 25°C, 1h, neat, 3eq bromofuran | 57 | 2 | 41 | 0 | 9 | 6 | 68 | 17 |

| | | | | | | | | | |
|---|---|---|---|---|---|---|---|---|---|
| X : total amount of the four isomers (IV) and (IVa) The four left columns give the propotion (mol%) of the 4 isomers of Diels-Alder adduct (IV and IVa can be endo or exo) A : amount of product A | | | | | | | | | |

## Claims

1. Process for the preparation of a 4,4'-dihalobenzophenone of formula (I): 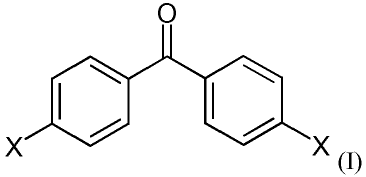 wherein X represents a halogen atom selected from the group consisting of fluorine, chlorine, bromine and iodine,
comprising carrying out the dehydration/aromatization of a compound of formula (VII): 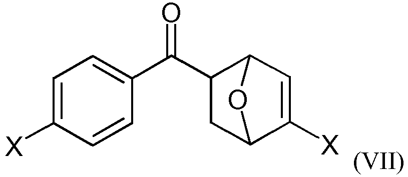 wherein X is as defined above.

2. Process according to claim 1, said process comprising the steps of:
a1) reacting a halofuran of formula (II) with a vinylhydrocarbylketone of formula (III): 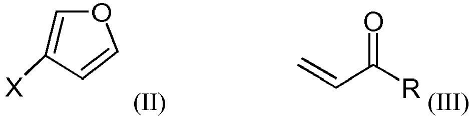 wherein X is the same as defined in claim 1, and R is a methyl radical,
thereby providing reaction products containing a cycloadduct compound of formula (IV): 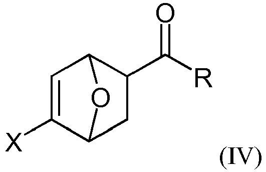 in which X and R are the same as defined above
a2) optionally further isolating said compound of formula (IV)
b) carrying out a dehydration/aromatization of said adduct compound of formula (IV), thereby providing reaction products containing a (p-halogeno)phenyl hydrocarbyl ketone of formula (V): 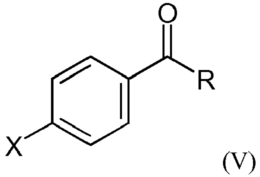 in which X and R are the same as defined above
c) carrying out an alpha-methylenation of said compound of formula (V), thereby providing reaction products containing a (p-halogeno)phenyl vinyl ketone of formula (VI): 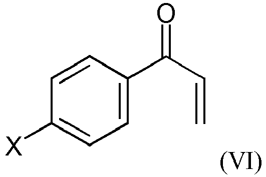 in which X is the same as defined above
d1) reacting said compound of formula (VI) with the same halofuran of formula (II) as defined above, thereby providing reaction products containing the cycloadduct compound of formula (VII): 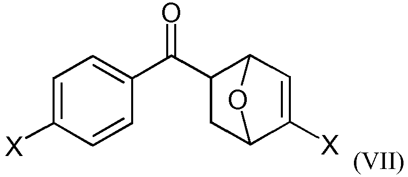 in which X is the same as defined above
d2) optionally further isolating said compound of formula (VII)
e) carrying out a dehydration/aromatization of said adduct compound of formula (VII), thereby providing reaction products containing said 4,4'-dihalobenzophenone of formula (I).

3. Process according to claim 2, wherein the dehydration/aromatization is carried out in the presence of an alkali methoxide, preferably sodium methoxide or potassium methoxide, or an alkali hydroxide, preferably sodium hydroxide or potassium hydroxide.

4. Process according to claim 2 or 3, wherein the dehydration/aromatization is carried out in the presence of DMSO or an alcohol.

5. Compound of formula (IV): 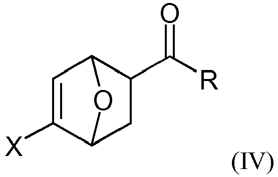 wherein X represents a halogen atom selected from the group consisting of fluorine, chlorine, bromine and iodine, and R is a hydrocarbyl radical.

6. Compound according to claim 5, wherein the halogen atom is selected from bromine and chlorine, preferably chlorine.

7. Compound according to claim 5 or 6, wherein R is a methyl radical.

8. Process for the preparation of a compound of formula (IV) as defined in any of claims 5 to 7, comprising reacting a halofuran of formula (II) as defined above with a vinylhydrocarbylketone of formula (III) in which R is the desired hydrocarbyl radical, and optionally further isolating compound of formula (IV).

9. Process according to claim 8, wherein the molar ratio of the halofuran of formula (II) to the vinylhydrocarbylketone of formula (III) is of at least 1/1.

10. Compound of formula (VII): 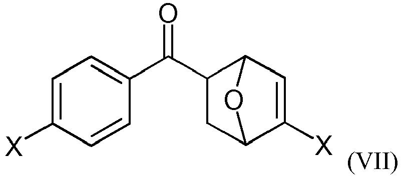 wherein X represents a halogen atom selected from the group consisting of fluorine, chlorine, bromine and iodine.

11. Compound according to claim 10, wherein the halogen atom is selected from bromine and chlorine, preferably chlorine.

12. Process for the preparation of a compound of formula (VII) as defined in claim 10 or 11, comprising reacting a halofuran of formula (II) with a (p-halogeno)phenyl vinyl ketone of formula (VI), said formulae being the same as defined above, and optionally further isolating compound of formula (VII).

13. Process according to claim 12, wherein the molar ratio of the halofuran of formula (II) to the (p-halogeno)phenyl vinyl ketone of formula (VI) is of at least 1/1.

14. Use of a compound of formula (IV) as defined in claim 7 or of a compound of formula (VII) as defined in claim 10 or 11 for the manufacture of a poly(aryl ether ketone), in particular poly(ether ether ketone).

## Patentansprüche

1. Verfahren zur Herstellung eines 4,4'-Dihalogenbenzophenons der Formel (I): 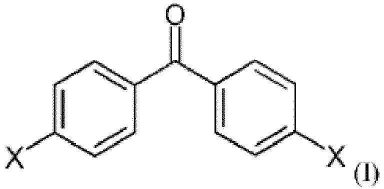 wobei X für ein Halogenatom aus der Gruppe bestehend aus Fluor, Chlor, Brom und Iod steht,
bei dem man die Dehydratisierung/Aromatisierung einer Verbindung der Formel (VII) 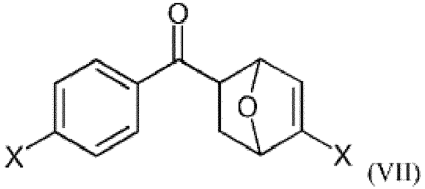 wobei X wie oben definiert ist,
durchführt.

2. Verfahren nach Anspruch 1, wobei man bei dem Verfahren folgende Schritte durchführt:
a1) Umsetzen eines Halogenfurans der Formel (II) mit einem Vinylhydrocarbylketon der Formel (III): 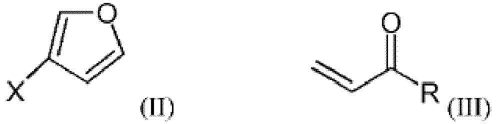 wobei X wie in Anspruch 1 definiert ist und R ein Methylrest ist,
wodurch man Reaktionsprodukte bereitstellt, die eine Cycloadduktverbindung der Formel (IV) enthalten: 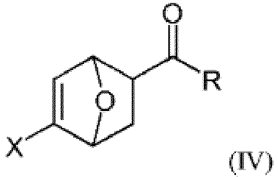 worin X und R wie oben definiert sind,
a2) gegebenenfalls weiteres Isolieren der Verbindung der Formel (IV),
b) Durchführen einer Dehydratisierung/Aromatisierung der Adduktverbindung der Formel (IV), wodurch man Reaktionsprodukte bereitstellt, die ein (p-Halogen)phenylhydrocarbylketon der Formel (V) enthalten: 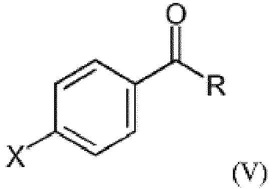 worin X und R wie oben definiert sind,
c) Durchführen einer alpha-Methylenierung der Verbindung der Formel (V), wodurch man Reaktionsprodukte bereitstellt, die ein (p-Halogen)phenylvinylketon der Formel (VI) enthalten: 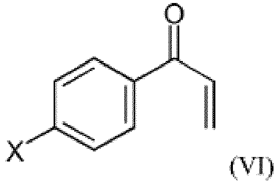 worin X wie oben definiert ist,
d1) Umsetzen der Verbindung der Formel (VI) mit demselben wie oben definierten Halogenfuran der Formel (II), wodurch man Reaktionsprodukte bereitstellt, die die Cycloadduktverbindung der Formel (VII) enthalten: 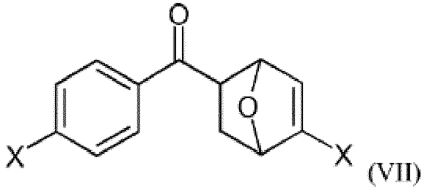 worin X wie oben definiert ist,
d2) gegebenenfalls weiteres Isolieren der Verbindung der Formel (VII),
e) Durchführen einer Dehydratisierung/Aromatisierung der Adduktverbindung der Formel (VII), wodurch man Reaktionsprodukte bereitstellt, die das 4,4'-Dihalogenbenzophenon der Formel (I) enthalten.

3. Verfahren nach Anspruch 2, wobei man die Dehydratisierung/Aromatisierung in Gegenwart von einem Alkalimethanolat, vorzugsweise Natriummethanolat oder Kaliummethanolat, oder einem Alkalihydroxid, vorzugsweise Natriumhydroxid oder Kaliumhydroxid, durchführt.

4. Verfahren nach Anspruch 2 oder 3, wobei man die Dehydratisierung/Aromatisierung in Gegenwart von DMSO oder einem Alkohol durchführt.

5. Verbindung der Formel (IV): 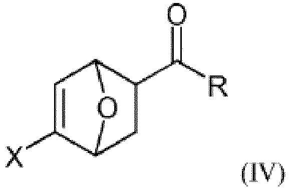 wobei X für ein Halogenatom aus der Gruppe bestehend aus Fluor, Chlor, Brom und Iod steht und R ein Hydrocarbylrest ist.

6. Verbindung nach Anspruch 5, wobei das Halogenatom aus Brom und Chlor, vorzugsweise Chlor, ausgewählt ist.

7. Verbindung nach Anspruch 5 oder 6, wobei R ein Methylrest ist.

8. Verfahren zur Herstellung einer Verbindung der Formel (IV) gemäß einem der Ansprüche 5 bis 7, bei dem man ein wie oben definiertes Halogenfuran der Formel (II) mit einem Vinylhydrocarbylketon der Formel (III) umsetzt, worin R der gewünschte Hydrocarbylrest ist, und die Verbindung der Formel (IV) gegebenenfalls weiter isoliert.

9. Verfahren nach Anspruch 8, wobei das Molverhältnis von dem Halogenfuran der Formel (II) zu dem Vinylhydrocarbylketon der Formel (III) mindestens 1/1 beträgt.

10. Verbindung der Formel (VII): 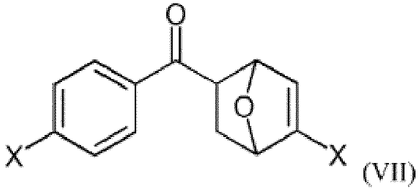 wobei X für ein Halogenatom aus der Gruppe bestehend aus Fluor, Chlor, Brom und Iod steht.

11. Verbindung nach Anspruch 10, wobei das Halogenatom aus Brom und Chlor, vorzugsweise Chlor, ausgewählt ist.

12. Verfahren zur Herstellung einer Verbindung der Formel (VII) gemäß einem der Ansprüche 10 oder 11, bei dem man ein Halogenfuran der Formel (II) mit einem (p-Halogen)phenylvinylketon der Formel (VI) umsetzt, wobei die Formeln wie oben definiert sind, und die Verbindung der Formel (VII) gegebenenfalls weiter isoliert.

13. Verfahren nach Anspruch 12, wobei das Molverhältnis von dem Halogenfuran der Formel (II) zu dem (p-Halogen)phenylvinylketon der Formel (VI) mindestens 1/1 beträgt.

14. Verwendung einer Verbindung der Formel (IV) gemäß Anspruch 7 oder einer Verbindung der Formel (VII) gemäß Anspruch 10 oder 11 zur Herstellung eines Poly(aryletherketons), insbesondere Poly(etheretherketon).

## Revendications

1. Procédé pour la préparation d'une 4,4'-dihalogénobenzophénone de formule (I) : 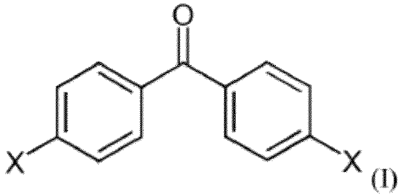 X représentant un atome d'halogène choisi dans le groupe constitué par fluor, chlore, brome et iode, comprenant la mise en œuvre de la déshydratation/aromatisation d'un composé de formule (VII) : 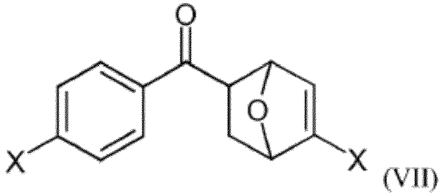 X étant tel que défini ci-dessus.

2. Procédé selon la revendication 1, ledit procédé comprenant les étapes de :
a1) mise en réaction d'un halogénofuranne de formule (II) avec une vinylhydrocarbylcétone de formule (III) : 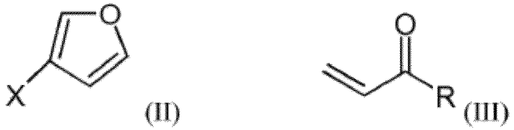 X étant le même que défini dans la revendication 1, et R étant un radical méthyle,
produisant ainsi des produits de réaction contenant un composé cycloadduit de formule (IV) : 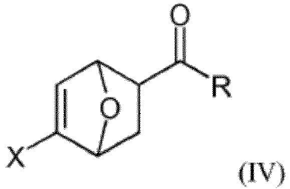 dans laquelle X et R sont les mêmes que définis ci-dessus
a2) éventuellement en outre isolement dudit composé de formule (IV)
b) mise en œuvre d'une déshydratation/aromatisation dudit composé adduit de formule (IV), fournissant ainsi des produits de réaction contenant une (p-halogéno)phénylhydrocarbylcétone de formule (V) : 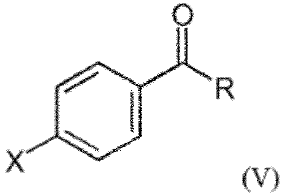 dans laquelle X et R sont les mêmes que définis ci-dessus
c) mise en œuvre d'une alpha-méthylénation dudit composé de formule (V), fournissant ainsi des produits de réaction contenant une (p-halogéno)phénylvinylcétone de formule (VI) : 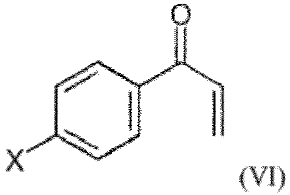 dans laquelle X est le même que défini ci-dessus
d1) mise en réaction dudit composé de formule (VI) avec le même halogénofuranne de formule (II) que défini ci-dessus, fournissant ainsi des produits de réaction contenant le composé cycloadduit de formule (VII) : 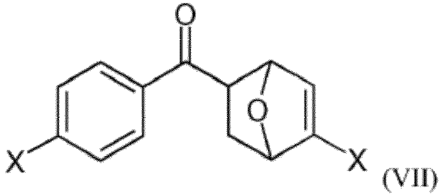 dans laquelle X est le même que défini ci-dessus
d2) éventuellement en outre isolement dudit composé de formule (VII)
e) mise en œuvre d'une déshydratation/aromatisation dudit composé adduit de formule (VII), fournissant ainsi des produits de réaction contenant ladite 4,4'-dihalogénobenzophénone de formule (I).

3. Procédé selon la revendication 2, la déshydratation/aromatisation étant mise en œuvre en présence d'un méthoxyde alcalin, préférablement le méthoxyde de sodium ou le méthoxyde de potassium, ou d'un hydroxyde alcalin, préférablement l'hydroxyde de sodium ou l'hydroxyde de potassium.

4. Procédé selon la revendication 2 ou 3, la déshydratation/aromatisation étant mise en œuvre en présence de DMSO ou d'un alcool.

5. Composé de formule (IV) : 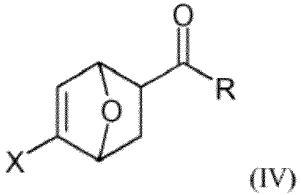 X représentant un atome d'halogène choisi dans le groupe constitué par fluor, chlore, brome et iode, et R étant un radical hydrocarbyle.

6. Composé selon la revendication 5, l'atome d'halogène étant choisi parmi brome et chlore, préférablement chlore.

7. Composé selon la revendication 5 ou 6, R étant un radical méthyle.

8. Procédé pour la préparation d'un composé de formule (IV) tel que défini dans l'une quelconque des revendications 5 à 7, comprenant la mise en réaction d'un halogénofuranne de formule (II) tel que défini ci-dessus avec une vinylhydrocarbylcétone de formule (III) dans laquelle R est le radical hydrocarbyle souhaité, et éventuellement en outre l'isolement du composé de formule (IV).

9. Procédé selon la revendication 8, le rapport molaire de l'halogénofuranne de formule (II) à la vinylhydrocarbylcétone de formule (III) étant d'au moins 1/1.

10. Composé de formule (VII) : 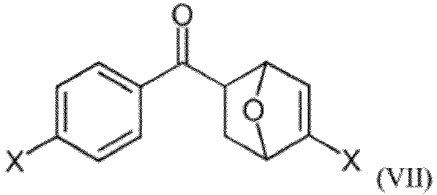 X représentant un atome d'halogène choisi dans le groupe constitué par fluor, chlore, brome et iode.

11. Composé selon la revendication 10, l'atome d'halogène étant choisi parmi brome et chlore, préférablement chlore.

12. Procédé pour la préparation d'un composé de formule (VII) tel que défini dans la revendication 10 ou 11, comprenant la mise en réaction d'un halogénofuranne de formule (II) avec une (p-halogéno)phénylvinylcétone de formule (VI), lesdites formules étant les mêmes que définies ci-dessus, et éventuellement en outre l'isolement du composé de formule (VII).

13. Procédé selon la revendication 12, le rapport molaire de l'halogénofuranne de formule (II) à la (p-halogéno)phénylvinylcétone de formule (VI) étant d'au moins 1/1.

14. Utilisation d'un composé de formule (IV) tel que défini dans la revendication 7 ou d'un composé de formule (VII) tel que défini dans la revendication 10 ou 11 pour la fabrication d'une poly(aryléthercétone), en particulier une poly(étheréthercétone) .
